# EUROPÄISCHE PATENTSCHRIFT

(11) **EP 0 624 073 B1**
(45) Veröffentlichungstag und Bekanntmachung des Hinweises auf die Patenterteilung: **27.09.1995**
(21) Anmeldenummer: 93902070.7
(22) Anmeldetag: 28.01.1993
(51) Int. Cl.: A61B 3/036

(54) **REFRAKTIONSGERÄT ZUR SUBJEKTIVEN BESTIMMUNG DER SPHÄRISCHEN UND ASTIGMATISCHEN SEHEIGENSCHAFTEN DES AUGES**
REFRACTION DEVICE FOR THE SUBJECTIVE DETERMINATION OF THE SPHERICAL AND ASTIGMATIC SIGHT PROPERTIES OF THE EYE
DISPOSITIF DE REFRACTION POUR LA DETERMINATION SUBJECTIVE DES PROPRIETES VISUELLES SPHERIQUES ET ASTIGMATIQUES DE L'OEIL

(30) Priorität: 01.02.1992 DE 4202902
(43) Veröffentlichungstag der Anmeldung: 17.11.1994
(73) Patentinhaber: KAMPPETER, Bernd, D-95447 Bayreuth (DE)
(72) Erfinder: KAMPPETER, Bernd, D-95447 Bayreuth (DE)
(74) Vertreter: LOUIS, PÖHLAU, LOHRENTZ & SEGETH
(86) Internationale Anmeldenummer: DE9300081
(87) Internationale Veröffentlichungsnummer: WO9314691

(56) Entgegenhaltungen:
- US-A- 3 240 548
- US-A- 3 811 756
- US-A- 3 927 933
- US-A- 4 385 813
- US-A- 4 943 162

## Beschreibung

Die Erfindung betrifft ein Refraktionsgerät zur subjektiven Bestimmung der sphärischen und astigmatischen Seheigenschaften des Auges mit einer Einrichtung zur Strahlenaufteilung in zwei Teilstrahlengänge, wodurch mindestens ein Sichtzeichen im Auge in zwei mit einem Blick erfaßbare Vergleichsbilder aufgeteilt wird, und mit einer dem jeweiligen Teilstrahlengang zugeordneten Optikeinrichtung, die zur Einstellung bestimmter optischer Eigenschaften in den beiden Teilstrahlengängen geeignet ist.

Ein solches Refraktionsgerät ist aus der DE-AS 1 155 615 bzw. aus der DE-Z "Der Augenoptiker", Heft 2, 1967, Seiten 7 bis 10 und 39 bekannt. Bei diesem bekannten Refraktionsgerät kommen als Optikeinrichtung Zylindergläser zur Anwendung, mit welchen die Vergleichsbilder des Sehzeichens nur um ± 0,25 Dioptrien unterschiedlich gemacht werden können. Weist das zu untersuchende Auge eine schlechte Sehkraft auf, so sind diese geringen Dioptrienunterschiede jedoch kaum wahrnehmbar. Ein weiterer Mangel besteht bei diesem bekannten Refraktionsgerät darin, daß bei der Zylinderachsenbestimmung miteinander Gläser verglichen werden, deren resultierende Zylinderachse dem Untersucher unbekannt sind, und deren astigmatischer Unterschied auch bei den höchsten Zylindern nur ± 0,25 Dioptrien beträgt. Außerdem verfügt dieses bekannte Refraktionsgerät über einen starren prismatischen Versatz zur Aufteilung eines Strahles in zwei Teilstrahlengänge und ist deshalb nur für die sog. 5-Meter-Visusstrecke als Zusatzgerät für einen handelsüblichen Phoropter geeignet. Dieser starre prismatische Versatz läßt nur eine Augen-Prüfung mit einem Sehzeichen der Sehschärfe 0,6 zu. Kleinere Sehzeichen führen dazu, daß die Vergleichsbilder zu weit auseinanderklaffen, und größere Sehzeichen führen zu einer Überlappung der Vergleichsbilder. Beides ist jedoch für einen exakten subjektiven Vergleich nachteilig. Ein weiterer nicht zu unterschätzender Mangel dieses bekannten Refraktionsgerätes zum Simultantest besteht darin, daß es zur Zylinderachsenbestimmung erforderlich ist, das Refraktionsgerät entsprechend zu verdrehen. Dadurch verlieren jedoch die Vergleichsbilder ihre Zuordnung, so daß die Kommunikation zwischen dem Patienten und der das Refraktionsgerät bedienenden Person beeinträchtigt bzw. erschwert ist. Außerdem ist durch den asymmetrischen Strahlengang bei diesem bekannten Refraktionsgerät ein Objekt bzw. Vergleichsbild um einige Zentimeter näher abgebildet als das andere Objekt bzw. Vergleichsbild. Ein weiterer Mangel besteht darin, daß die Untersuchungsstategie bei diesem Refraktionsgerät genau so schwer erlernbar ist, wie bei den üblichen Refraktionsgeräten, weil eine Benutzerführung mittels Computer nicht möglich ist.

Aus der US-A 4 943 162 ist ein Refraktionsgerät mit einem Zylinderkompensator bekannt, das ein Zusatzgerät für herkömmliche Phoropter bildet. Dieses Refraktionsgerät soll einen Patienten in die Lage versetzen, seinen astigmatischen Fehler durch das Bedienen zweier hintereinander angeordneter Sims-Gläser, d.h. hintereinander angeordneter Zylindergläser, selbst einzustellen. Mit diesem bekannten Refraktionsgerät ist es nicht möglich, zwei Vergleichsbilder gleichzeitig miteinander zu vergleichen, um auf diese Weise eine subjektive Bestimmung der Seheigenschaften des Auges durchzuführen. Das bedeutet jedoch, daß die mit diesem Refraktionsgerät durchgeführte Refraktionsbestimmung ohne Vergleichsbilder bei größenordnungsmäßig 80 % des normalen Augenarzt-Publikums unmöglich sein dürfte.

Die US-A 4 529 280 offenbart ein Nahsichtgerät, das jedoch nicht dazu in der Lage ist, einen simultanen Bildvergleich durchzuführen. Eine Sehschärfenbestimmung mit variablen Sehzeichen ist dort nicht möglich. Die Ermittlung der Refraktion erfolgt entsprechend einer Beurteilung des Patienten, der jedoch simultan keine Vergleichsbilder zur Verfügung hat. Das führt jedoch nach allen Erfahrungen der Ophthalmologie bei einem Großteil der Patienten zu falschen Ergebnissen. Ophthalmologische Untersuchungsmethoden sind bspw. auch im DE-B von W. Straub, Band II, Ferdinand-Enke-Verlag Stuttgart 1976, beschrieben.

Die US-A 4 834 527 beschreibt ein Gerät zur Abstandssicherung zwischen Patient und ophthalmologischem Gerät nach dem Prinzip des sog. Non-Contact-Tonometers.

Der Erfindung liegt die Aufgabe zugrunde, ein Refraktionsgerät der eingangs genannten Art zu schaffen, das einfach ausgebildet und einfach bedienbar ist, und mit welchem eine wesentliche Verbesserung der Brillenbestimmung erzielbar ist.

Diese Aufgabe wird bei einem Refraktionsgerät der eingangs genannten Art erfindungsgemäß dadurch gelöst, daß in jedem der beiden Teilstrahlengänge als Optikeinrichtung ein Zylinderkompensator angeordnet ist, der mindestens ein Pluszylinderglas und mindestens ein Minuszylinderglas aufweist, wobei das/jedes im entsprechenden Teilstrahlengang vorgesehene Plus- und Minuszylinderglas zur Zylinderhöheneinstellung mittels einer zugehörigen ersten Antriebseinrichtung gegeneinander um die entsprechende Teilstrahlengangachse verdrehbar sind, und wobei das/jedes Plus- und das/jedes Minuszylinderglas jedes Teilstrahlengangs zur entsprechenden Zylinderachseneinstellung mittels einer zugehörigen zweiten Antriebseinrichtung um die entsprechende Teilstrahlengangachse jeweils gemeinsam verdrehbar sind. Weist das Pluszylinderglas z.B. +4 Dioptrien und das Minuszylinderglas -4 Dioptrien auf, so lassen sich in jedem der beiden Teilstrahlengänge sämtliche Zylinderwerte zwischen +4 und -4 Dioptrien simulieren. Mit einem derartig ausgestatteten Refraktionsgerät ist es möglich, 8 Dioptrien Astigmatismus zu korrigieren. Dieser Dioptrienbereich deckt mehr als 99 % aller Brillenrefraktionen ab. Desgleichen ist es mit dem erfindungsgemäßen Refraktionsgerät einfach möglich, sämtliche Zylinderachsen zwischen 0 und 180 Winkelgrad in jedem der beiden Teilstrahlengänge direkt und definiert zu erzeugen und miteinander zu vergleichen. Sie können den variablen Sehleistungen der Patienten angeglichen werden. Die Verdrehung jedes der beiden Zylinderkompensatoren sowie die Verdrehung der beiden Zylindergläser des entsprechenden Zylinderkompensators ist erfindungsgemäß bspw. mittels Schrittmotoren jeweils z.B. in Winkelschritten möglich, die bei 1 Winkelgrad liegen können. Das bedeutet jedoch, daß mit dem erfindungsgemäßen Refraktionsgerät die sphärischen und astigmatischen Seheigenschaften des Auges eines Patienten sehr genau bestimmbar sind. Eine vergleichbare Genauigkeit ist bei manuell bedienbaren Zylinderkompensatoren nicht erzielbar.

Wie bereits erwähnt worden ist, kann beim erfindungsgemäßen Refraktionsgerät das Pluszylinderglas z.B. +4 Dioptrien und das Minuszylinderglas jedes der beiden Zylinderkompensatoren -4 Dioptrien oder mehr aufweisen. Um einen derartigen Dioptrien-Bereich abzudecken, sind beim eingangs erwähnten, bekannten Refraktionsgerät nach Biessels, wie es in der erwähnten DE-AS 1 155 615 beschrieben worden ist, durch den 0,25 Dioptrien-Abstand zweiunddreißig Gläser erforderlich. Demgegenüber sind erfindungsgemäß nur zwei Gläser erforderlich. Mit insgesamt vier Gläsern können bei Zylinderkompensatoren alle vier Gläser zwischen 0 und 8 Dioptrien stufenlos in sämtlichen Achslagen direkt miteinander verglichen werden. Bei dem oben genannten Gerät nach Biessel's können trotz des Einsatzes von zweiunddreißig Gläsern nur Gläser mit 0,5 Dioptrien Unterschied und Achslagen mit 90° miteinander verglichen werden.

Die Strahlenaufteilungseinrichtung kann erfindungsgemäß zwei Paare von jeweils zueinander mindestens annähernd parallelen Spiegeln aufweisen, die von der Patientenseite wegdivergieren, wobei die Spiegelpaare zueinander mindestens annähernd senkrecht orientiert sind. Die besagten Spiegelpaare können durch geeignete Prismen ersetzt werden.

Bei einem Refraktionsgerät der oben genannten Art kann das eine Paar Spiegel ortsfest und ein Spiegel des zweiten Paares Spiegel zur Ausrichtung der beiden Teilstrahlengänge auf ein einziges Sichtzeichen mittels einer dritten Antriebseinrichtung in seinem Winkel relativ zum anderen Spiegel des zweiten Paares Spiegel verstellbar sein. Eine solche Ausbildung des erfindungsgemäßen Refraktionsgerätes erfordert zur simultanen Beobachtung nur ein einziges Sichtzeichen. Durch die Möglichkeit, mit Hilfe der genannten dritten Antriebseinrichtung den zugehörigen Spiegel des zweiten Paares Spiegel wunschgemäß verstellen zu können, ist das erfindungsgemäße Refraktionsgerät in vorteilhafter Weise für Sehzeichen beliebiger Größe gleich gut geeignet, weil durch entsprechende Neigungsverstellung des mit der dritten Antriebseinrichtung verbundenen Spiegels Überlappungen der beiden simultan gegebenen Vergleichsbilder bzw. ein zu weites Auseinanderklaffen derselben vermieden werden.

Beim erfindungsgemäßen Refraktionsgerät als Zusatzgerät zu einem konventionellen Phoropter kann zum entsprechenden Sphäre-Vergleich eine mit sphärischen Gläsern und mit einem Verschluß versehene Scheibe vorgesehen sein, die auf der vom Patienten abgewandten Seite nach den beiden Zylinderkompensatoren angeordnet und mittels einer vierten Antriebseinrichtung um eine zu den beiden Teilstrahlengängen parallele Drehachse drehbar ist. Mit einer solchen Ausbildung des Refraktionsgerätes ist es mit Hilfe der vierten Antriebseinrichtung möglich, z.B. den einen der beiden Teilstrahlengänge zu verschließen oder in beiden Teilstrahlengängen unterschiedliche sphärische Gläser anzuordnen, um sphärisch unterschiedliche Vergleichsbilder zu erzeugen.

Refraktionsgeräte der oben beschriebenen Art können einen auf der dem Patienten zugewandten Seite vorgesehenen, eine Geräteeintrittspupille bildenden Montageabschnitt zur Befestigung des Refraktionsgerätes als Vorsatzgerät an einem an sich bekannten Phoropter aufweisen. Ein solches Refraktionsgerät kann also mit jedem handelsüblichen Phoropter kombiniert werden, um auf diese Weise alle drei Parameter der subjektiven Refraktion, d.h. die Sphäre, die Zylinderhöhe und die Achslage des Zylinders im gleichzeitigen Vergleich zu ermitteln. Diese drei Parameterwerte beeinflussen sich gegenseitig, wobei diese gegenseitige Beeinflussung mit Hilfe des erfindungsgemäßen Refraktionsgerätes einfacher und zeitsparender handhabbar ist,ohne die Bestimmung der besagten drei Parameterwerte zu beeinträchtigen. Erheblich unterschiedliche Gläser gleichen sphärischen Äquivalents, z.B. -1,0 sph. mit +/- 0 -2,0/90°, können direkt und gleichzeitig miteinander verglichen werden, was bisher mit keiner Methode möglich war. Mißverständnisse zwischen Untersucher und Patient werden vermieden. Bei der konventionellen Untersuchung besteht ein Interessenkonflikt zwischen Refraktionist und Patient. Der Patient möchte die an der Sehgrenze angesiedelten Sehzeichen möglichst lange ansehen und entziffern. Der Arzt muß aber die Vergleichsgläser möglichst schnell nacheinander anbieten, damit der zu vergleichende Seheindruck nicht aus dem Kurzzeitgedächtnis des Patienten entschwindet.

Zur Ausbildung eines eigenständigen Gerätes als kompletter Phoropter mit Simultanvergleich für die 5-Meter-Sehteststrecke können auf der dem Patienten zugewandten Seite des erfindungsgemäßen Refraktionsgerätes zwei zueinander parallele Scheiben mit im gemeinsamen Strahlengang vor der Geräteeintrittspupille angeordneten sphärischen Gläsern vorgesehen sein, wobei die sphärischen Gläser der einen Scheibe einen im Vergleich zum Dioptrienabstand der sphärischen Gläser der zweiten Scheibe großen Dioptrienabstand aufweisen. Die beiden Scheiben sind zweckmäßigerweise mittels einer fünften Antriebseinrichtung verdrehbar. Hierbei können die sphärischen Gläser der einen Scheibe z.B. voneinander einen Abstand von jeweils drei Dioptrien und die sphärischen Gläser der zweiten Scheibe jeweils einen Abstand von 0,25 Dioptrien aufweisen. Durch diese Scheiben können bei Einsatz von z.B. elf Gläsern im 0,25 Dioptrien Abstand und elf Gläsern im 3 Dioptrien Bereich durch Addition sämtliche sphärischen Gläser zwischen +18,0 und -17,75 Dioptrien hergestellt werden. Die unterschiedlichen Zylindergrößen und Achsen werden durch die vorgeschriebenen Zylinderkompensatoren im jeweiligen Strahlengang erzeugt. Die unterschiedlichen sphärischen Zustände werden wie beim zuvor beschriebenen Vorsatzgerät durch eine Scheibe auf der dem Patienten abgewandten Seite des Gerätes zugegeben. Gearbeitet wird mit einem Sehzeichengerät, das die Sehzeichen im 5-Meter-Abstand abbildet.

Eine weitere Möglichkeit ergibt sich erfindungsgemäß durch ein Gerät zur simultanen Refraktionsbestimmung im Nahbereich. Dieses Refraktionsgerät kann in vorteilhafter Weise kleinvolumig ausgebildet sein, da es keinen größeren Platzbedarf bzw. keine 5-Meter-Sehstrecke erfordert. Mit dem genannten erfindungsgemäßen Refraktionsgerät ist es möglich, einen Patienten an der Geräteeintrittspupille um einen bestimmten Betrag zu myopisieren, d.h. der Fernpunkt des Patienten kann durch Vorsetzen von Plusgläsern in die Nähe verlegt werden. Bspw. kann der Fernpunkt eines Normalsichtigen durch Vorsetzen von +10 Dioptrien auf 10cm eingestellt werden. Wird ein Sehzeichen zum Beispiel bis auf 5 cm nach vorne bewegt, bis der Patient es scharf fokusiert erkennt, entspricht es einer sphärischen Refraktion des Patientenauges von -10 Dioptrien. Umgekehrt muß es dann z.B. von 10 cm bis auf 20 cm bis zur Fokusierung entfernt werden, um einer Refraktion des Auges von +5 Dioptrien zu entsprechen. Sollte wegen hoher Myopie oder Hyperopie dieser Bereich nicht ausreichen, werden entsprechende Vorsatzgläser in der Scheibe gewählt. Zur weiteren Verdeutlichung mag das nachfolgende Beispiel dienen:

Vorsatzglas +/-0 deckt bei einem Abstand von 5-20 cm den Bereich von -20,0 bis -5,0 sphärisch ab. Das Vorsatzglas +20,0 sph. würde z.B. den Bereich von +/-0 sph. bis +15,0 Dptr. bei gleicher Sehstrecke abdecken.

Eine andere Ausbildung des erfindungsgemäßen Refraktionsgerätes ist dadurch gekennzeichnet, daß die Einrichtung zur Strahlenaufteilung zwei hintereinander angeordnete Paare von jeweils parallelen Spiegeln aufweist, wobei der eine Spiegel des einen Spiegelpaares ein halbdurchlässiger Spiegel ist, hinter dem der erste Spiegel des zweiten Spiegelpaares zum halbdurchlässigen Spiegel des ersten Spiegelpaares mindestens annähernd senkrecht ausgerichtet ist und die zweiten Spiegel der beiden Spiegelpaare gegeneinander seitlich versetzt sind. Ein solches Refraktionsgerät kann zur simultanen Refraktionsbestimmung im Nahbereich oder zur simultanen Refraktionsbestimmung mit Sphärenbestimmung nach dem Prinzip des astronomischen Fernrohres vorgesehen sein. Im zuerst genannten Fall, d.h. bei einem Refraktionsgerät zur simultanen Refraktionsbestimmung im Nahbereich kann auf der vom Patienten abgewandten Seite der Strahlenaufteilungseinrichtung eine Sehzeicheneinrichtung mit zwei Sehzeichenelementen vorgesehen sein, von welchen jedes in einem zugehörigen Teilstrahlengang angeordnet und mittels einer zugehörigen sechsten Antriebseinrichtung in bezug auf die Einrichtung zur Strahlenaufteilung linear verstellbar ist. Bei einem solchen Refraktionsgerät ist es zweckmäßig, wenn in jedem Teilstrahlengang der entsprechende mit der ersten und der zweiten Antriebseinrichtung verbundene Zylinderkompensator zwischen der Strahlenaufteilungseinrichtung und der Sehzeicheneinrichtung ortsunveränderlich angeordnet ist und wenn auf der dem Patienten zugewandten Seite vor der Strahlenaufteilungseinrichtung eine Bereichswählscheibe mit sphärischen Gläsern unterschiedlicher Dioptrienzahlen vorgesehen ist, die wahlweise nacheinander mittels einer siebenten Antriebseinrichtung im Lichtstrahl der Eintrittspupillen anordenbar bzw. austauschbar sind und zur Bereichserweiterung vorgesetzt werden können. Mit einem solchen Refraktionsgerät kann der Patient durch die besagte Bereichswählscheibe an der Geräteeintrittspupille um einen bestimmten Betrag myopisiert werden. Die abweichende sphärische Refraktion wird durch entsprechende lineare Verstellung der beiden Sehzeichenelemente, d.h. durch Vor- oder Zurückbewegen der besagten Sehzeichenelemente bestimmt. Beim kurzsichtigen Patienten werden die Sehzeichenelemente näher und beim übersichtigen Patienten werden die Sehzeichenelemente entsprechend weiter weg bewegt. Der Verstellweg entspricht der sphärischen Refraktion des Patienten. Der Meßbereich des Refraktionsgerätes kann durch die genannte Bereichswählscheibe mit den entsprechenden voneinander verschiedenen Plusgläsern dem Meßbereich von z.B. ±25 Dioptrien und dem Sehzeichenweg von z.B. 5 bis 25 cm angepaßt werden. In vorteilhafter Weise werden mit einem solchermaßen ausgebildeten Refraktionsgerät anstelle von ca. 160 Gläsern bei Refraktionen mit einem üblichen Probiergestell erfindungsgemäß nur noch 4 bis 6 Gläser benötigt. Durch unterschiedliches Bewegen der Sehzeichenelemente werden die unterschiedlichen sphärischen Zustände in beiden Strahlengänge erzeugt. Die Sehzeichen, bei welchen es sich z.B. um Zahlen oder Buchstaben handelt,werden bspw. auf zwei Trommeln symmetrisch in beiden Strahlengängen übereinander abgebildet. Die entstehenden Abbildungsgrößenunterschiede im Auge durch Näherkommen der Sehzeichenelemente werden durch Drehen der Trommeln mit kleiner werdenden Sehzeichen ausgeglichen.

Ein Freisichtgerät, d.h. ein Gerät mit feststehenden Sehzeichen im Nahbereich, zur simultanen Refraktionsbestimmung mit Sphärenbestimmung nach dem Prinzip des astronomischen Fernrohres wird beim erfindungsgemäßen Refraktionsgerät dadurch erreicht, daß in jedem Teilstrahlengang der entsprechende, mit der ersten und der zweiten Antriebseinrichtung verbundene Zylinderkompensator zwischen zwei Sammellinsen und zu diesen und in bezug auf das zugehörige Sehzeichenelement in einem entsprechenden Fernrohrteilsystem ortsunveränderlich vorgesehen ist, wobei jedes der beiden Fernrohrteilsysteme mittels einer zugehörigen achten Antriebseinrichtung linear entlang dem entsprechenden Teilstrahlengang relativ zu einer im entsprechenden Teilstrahlengang auf der vom Patienten abgewandten Seite der Strahlenaufteilungseinrichtung ortsfest angeordneten Sammellinse verstellbar ist. Die Ausbildung als Freisichtgerät hat dabei den weiteren ganz erheblichen Vorteil, daß eine Geräteakkomodation vermieden wird. Zu diesem Zweck ist auf der dem Patienten zugewandten Seite der Strahlenaufteilungseinrichtung zweckmäßigerweise ein halbdurchlässiger Spiegel schräg orientiert vorgesehen.

Als zweckmäßig hat es sich erwiesen, wenn bei den Refraktionsgeräten der oben genannten Art jede der beiden Sehzeicheneinrichtungen unterschiedlich große Sehzeichen aufweist und mittels einer zugehörigen neunten Antriebseinrichtung relativ zum entsprechenden Teilstrahlengang verstellbar ist. Bei den besagten Sehzeichen kann es sich um Buchstaben und/oder Zahlen handeln. Die Sehzeicheneinrichtungen sind - wie bereits erwähnt worden ist - zweckmäßigerweise als Trommeln ausgebildet, auf deren Mantelfläche die Sehzeichen in einer Drehrichtung größer bzw. kleiner werdend vorgesehen sind. Bei der subjektiven Refraktion sollte man mit dem gerade noch erkennbaren Sehzeichen prüfen, da es dem Patienten leichter fällt, zwei Gläser als besser oder schlechter zu unterscheiden, wenn er mit dem einen das Sehzeichen noch erkennt, es mit dem anderen aber nicht mehr entziffern kann.

Ein einfach zu handhabendes bzw. einfach zu bedienendes, nach der Gabelungsmethode arbeitendes Refraktionsgerät ergibt sich, wenn die beiden ersten und/oder die beiden zweiten und/oder die dritte und/oder die vierte und/oder die fünfte und/oder die beiden sechsten und/oder die siebente und/oder die beiden achten und/oder die beiden neunten Antriebseinrichtungen mit einem Microcomputer verbundene Schrittmotoren sind. Mittels der besagten Schrittmotoren sind die entsprechenden Verstellungen in Winkelschritten von z.B. 1 Winkelgrad und damit sehr genau möglich. Die Sphäre und die Zylindergröße der miteinander zu vergleichenden Gläser läßt sich so quasi stufenlos erzeugen. Bei schlechter Sehleistung wird ein großes Sehzeichen gewählt, und dieses solange verkleinert, bis die Sehgrenze erreicht ist. Anschließend werden große sphärische Gläser miteinander verglichen und immer wenn eine Sehverbesserung durch ein Glas eintritt, das Sehzeichen verkleinert, bis die Sehgrenze erreicht wird. Danach werden sphärische Gläser angeboten, die im halben Abstand vom zuvor besseren Glas entfernt liegen. Durch Befragen des Patienten erhält man so in mehreren Schritten die wahre sphärische Refraktion des Patienten, indem man sich durch Gabelung der Gläser und Nachstellung des Sehzeichens der optimalen Sehschärfe des Patienten annähert. In gleicher Weise geschieht dies bei Zylinder und Zylinderachse.

Durch den Microcomputer ist die mit dem erfindungsgemäßen Refraktionsgerät durchführbare subjektive Refraktionsbestimmung standardisierbar und so gestaltbar, daß auch unqualifiziertes Bedienungspersonal eine exakte subjektive Brillenbestimmung durchführen kann. Ggf. kann ein Patient sogar seine Refraktion selbst bestimmen, indem er am Computer zwei Tasten bedient, die einer Verbesserung im oberen oder unteren Strahlengang zugeordnet sind. Das bedeutet jedoch, daß das erfindungsgemäße Refraktionsgerät nicht nur für Erstweltländer sondern auch für Zweit- oder Drittweltländer zur exakten subjektiven Refraktionsbestimmung der verschiedensten Patienten gleich gut geeignet ist. Durch den Einsatz eines Microcomputers und durch die mit diesem zusammengeschalteten Schrittmotoren als Antriebseinrichtungen wird ein Refraktionsgerät zur Verfügung gestellt, bei welchem die Bedienungsperson nur noch ca. zehn- bis zwanzigmal an den Patienten dieselbe Frage stellen muß, ob z.B. das obere oder untere Bild schärfer gesehen wird. Die entsprechende Antwort wird in den Microcomputer eingegeben, wozu dieser bspw. mit einer Tastatur verbunden ist. Der Microprozessor errechnet dann die entsprechende subjektive Refraktion, wobei das erzielte Ergebnis wesentlich besser ist als das mit konventionellen Methoden erzielte Refraktionsergebnis. Selbstverständlich ist mit dem erfindungsgemäßen Refraktionsgerät auch die klassische Methode mit Glasvergleich zweier Gläser nacheinander möglich und an Stelle der Gabelungsmethode die klassische Kreuzzylindermethode anwendbar. Hier wird sogar das früher unerreichbare Ziel verwirklicht, zwei Gläser nacheinander anzubieten und zum Glaswechsel keine Zeit zu vergeuden: Dies ist z.B. dadurch möglich, daß die beiden Gläser im jeweiligen Strahlengang voreingestellt sind und nur durch An- und Ausschalten von Lichtquellen die Vergleichsgläser nacheinander ohne Verzögerung dargeboten werden.

Weitere Einzelheiten, Merkmale und Vorteile ergeben sich aus der nachfolgenden Beschreibung von in der Zeichnung schematisch dargestellten Ausführungsbeispielen des erfindungsgemäßen Refraktionsgerätes. Es zeigen:
- Fig. 1: eine erste Ausführungsform des Refraktionsgerätes, das als Vorsatzgerät für einen handelsüblichen Phoropter dienen kann,
- Fig. 2: schematisch ein Refraktionsgerät für die 5m-Sehstrecke, das die sphärische Refraktion mit zwei mit sphärischen Gläsern bestückten Scheiben erzeugt, wobei die Geräteteile 14, 20, 52 denen der Fig. 1 entsprechen.
- Fig. 3: eine Ausbildung des Refraktionsgerätes zur simultanen Refraktionsbestimmung im Nahbereich mit zwei Sichtzeichen, und
- Fig. 4: eine Ausbildung des Refraktionsgerätes als Freisichtgerät zur simultanen Refraktionsbestimmung mit Sphärenbestimmung nach dem Prinzip des astronomischen Fernrohres, mit zwei Sichtzeichen.

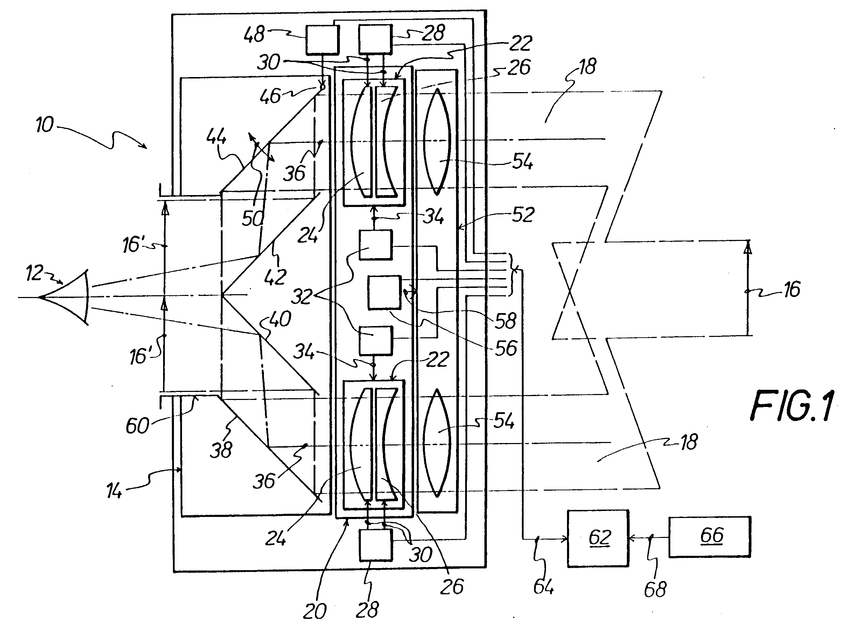

Fig. 1 zeigt schematisch eine erste Ausbildung des Refraktionsgerätes 10 zur subjektiven Bestimmung der sphärischen und astigmatischen Seheigenschaften eines Auges 12 eines Patienten. Das Refraktionsgerät 10 weist eine Strahlenaufteilungseinrichtung 14 auf, durch welche ein einziges Sichtzeichen 16, bei dem es sich bspw. um einen Buchstaben oder um eine Zahl handeln kann, in zwei Teilstrahlengänge 18 aufgeteilt wird. Das Sichtzeichen 16 wird durch einen Sehzeichenprojektor im 5m-Abstand erzeugt. Durch die Strahlenaufteilungseinrichtung 14 wird das Sichtzeichen 16 im Auge 12 des Patienten also in zwei Sichtzeichen 16' aufgeteilt, die mit einem Blick gleichzeitig erfaßbar sind, d.h. die simultan erfaßbare Vergleichsbilder darstellen. Mit der Bezugsziffer 20 ist durch einen Block eine Optikeinrichtung bezeichnet, die auf der vom Patienten 12 abgewandten Seite der Strahlenaufteilungseinrichtung 14 vorgesehen und die den beiden Teilstrahlengängen 18 zugeordnet ist. Die Optikeinrichtung 20 weist zwei Zylinderkompensatoren 22 auf, die in den Teilstrahlengängen 18 angeordnet sind. Jeder der beiden Zylinderkompensatoren 22 weist ein Pluszylinderglas 24 und ein Minuszylinderglas 26 auf. Das Pluszylinderglas 25 und das Minuszylinderglas 26 jedes Zylinderkompensators 22 sind mit einer ersten Antriebseinrichtung 28 wirkverbunden, was durch die Pfeile 30 angedeutet ist. Mit Hilfe der ersten Antriebseinrichtung 28 ist es möglich, das Pluszylinderglas 24 und das Minuszylinderglas 26 des zugehörigen Zylinderkompensators 22 voneinander unabhängig in bezug zueinander zu verdrehen, um auf diese Weise eine gewünschte Zylinderhöheneinstellung vorzunehmen. Jeder der beiden Zylinderkompensatoren 22 ist außerdem mit einer zugehörigen zweiten Antriebseinrichtung 32 wirkverbunden, was durch die Pfeile 34 angedeutet ist. Mit Hilfe der zweiten Antriebseinrichtung 32 ist es möglich, den zugehörigen Zylinderkompensator 22 insgesamt um die entsprechende Teilstrahlengangachse 36 zu verdrehen, um eine gewünschte Zylinderachseneinstellung vorzunehmen.

Die Strahlenaufteilungseinrichtung 14 weist ein erstes Paar Spiegel 38 und 40 auf, die zueinander parallel ausgerichtet ortsfest vorgesehen sind. Ein zweites Paar Spiegel 42 und 44 ist zueinander parallel und zum ersten Spiegelpaar 38, 40 divergierend bzw. mindestens annähernd senkrecht ausgerichtet vorgesehen. Der Spiegel 42 ist ortsfest angeordnet und der Spiegel 44 ist um eine Drehachse 46 mittels einer dritten Antriebseinrichtung 48 relativ zum Spiegel 42 verschwenkbar. Diese Verschwenkbarkeit ist durch den bogenförmigen Doppelpfeil 50 angedeutet.

Auf der vom Patienten abgewandten Seite der Optikeinrichtung 20 ist eine Scheibe 52 vorgesehen, die sphärische Gläser 54 und einen Verschluß aufweist, um in den beiden Teilstrahlengängen 18 entsprechende unterschiedliche sphärische Gläser 54 bzw. im einen oder anderen Teilstrahlengang 18 den besagten Verschluß anordnen zu können. Zu diesem Zweck ist die Scheibe 52 mit einer vierten Antriebseinrichtung 56 verbunden, was durch den Pfeil 58 angedeutet ist.

Das Refraktionsgerät 10 ist auf der dem Patienten zugewandten Seite mit einem Montageabschnitt 60 versehen, welcher eine Geräteeintrittspupille bildet. Mit diesem Montageabschnitt 60 kann das Refraktionsgerät 10 als Vorsatzgerät an einem an sich bekannten Phoropter temporär festgelegt werden.

Die Scheibe 52 mit den sphärischen Gläsern 54 dient zur Erzeugung einer unterschiedlichen Sphäreneinstellung, so daß es mit Hilfe der erwähnten Scheibe 52 und mit Hilfe der Optikeinrichtung 20 und der Benutzung der sphärischen Gläser des vorgeschalteten, nicht gezeichneten, konventionellen Phoropters möglich ist, vorzugsweise unter Zuhilfenahme einer computergesteuerten Strategie die drei Parameter der subjektiven Refraktion Sphäre, Zylinderhöhe und Zylinderachslage genau zu bestimmen. Die genaue Bestimmung der besagten drei Parameter der subjektiven Refraktion ist dadurch möglich, daß die Antriebseinrichtungen 28, 32, 48 und 56 mit einem Microcomputer 62 verbunden sind, was durch den Doppelpfeil 64 schematisch angedeutet ist. Mit dem Microcomputer 62 ist eine Tastatur 66 verbunden, was durch den Pfeil 68 angedeutet ist. Durch den Einsatz des Microcomputers 62 mit der Tastatur 66 und durch die Verstellbarkeit der Komponenten mit Hilfe der mit dem Microcomputer 62 verbundenen Antriebseinrichtungen 28, 32, 48 und 56 ergibt sich der Vorteil, daß die das Refraktionsgerät 10 bedienende Person dem Patienten nur noch ca. zehn- bis zwanzigmal die gleiche Frage zu stellen hat, ob das obere oder das untere Bild 16' schärfer ist. Die entsprechende Antwort wird dann mit Hilfe der Tastatur 66 in den Microcomputer 62 eingegeben. Durch entsprechende Programmierung des Microcomputers 62 ist es möglich, aus diesem dann unverzüglich die richtigen Parameter Sphäre, Zylinderhöhe und Zylinderachslage eines Patientenauges auszugeben, so daß ein Optiker die passenden Augengläser anfertigen kann.

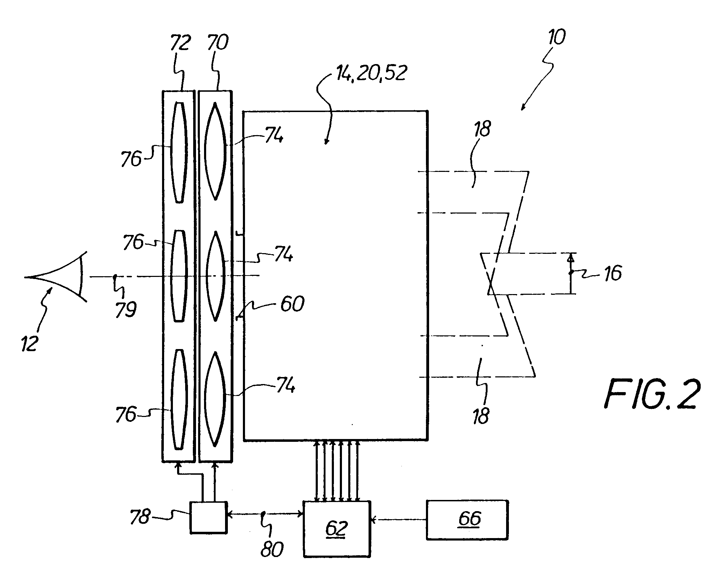

Fig. 2 zeigt schematisch eine Ausbildung des Refraktionsgerätes 10 für ein einziges Sichtzeichen 16, wobei das Refraktionsgerät 10 ähnlich dem in Fig. 1 schematisch dargestellten Refraktionsgerät 10 ausgebildet ist und auf alle Einzelheiten desselben verzichtet worden ist. Es sind nur die beiden divergierenden Teilstrahlengänge 18 auf der vom Patienten abgewandten Seite sowie der als Geräteeintrittspupille ausgebildete Montageabschnitt 60 auf der dem Patienten zugewandten Seite des Refraktionsgerates angedeutet. Desgleichen sind in Fig. 2 der Microcomputer 62 und die mit dem Microcomputer 62 verbundene Tastatur 66 schematisch dargestellt. Auf der dem Patienten (Auge 12) zugewandten Seite der Geräteeintrittspupille 60 sind zwei zueinander parallele Scheiben 70 und 72 mit sphärischen Gläsern 74 und 76 vorgesehen, um ein eigenständiges Gerät für die 5-Meter-Visus-Strecke auszubilden. Die sphärischen Gläser 74 der Scheibe 70 weisen einen Dioptrienabstand auf, der größer ist als der Dioptrienabstand der sphärischen Gläser 76 der zweiten Scheibe 72. Bspw. beträgt der Dioptrienabstand der sphärischen Gläser 74 drei Dioptrien, während der Dioptrienabstand der sphärischen Gläser 76 nur 0,25 Dioptrien beträgt. Die Scheiben 70 und 72 sind mittels einer fünften Antriebseinrichtung 78 verstellbar, um im Strahlengang 80 der Geräteaustrittspupille 60 jeweils eines der sphärischen Gläser 74 mit einem der sphärischen Gläser 76 geeignet anzuordnen, d.h. zu kombinieren. Die fünfte Antriebseinrichtung 78, bei der es sich wie bei den Antriebseinrichtungen 28, 32, 48 und 56 (sh. Fig. 1) vorzugsweise um einen elektrischen Schrittmotor handelt, ist mit dem Microcomputer wirkverbunden, was in Fig. 2 durch den Doppelpfeil 80 verdeutlicht ist.

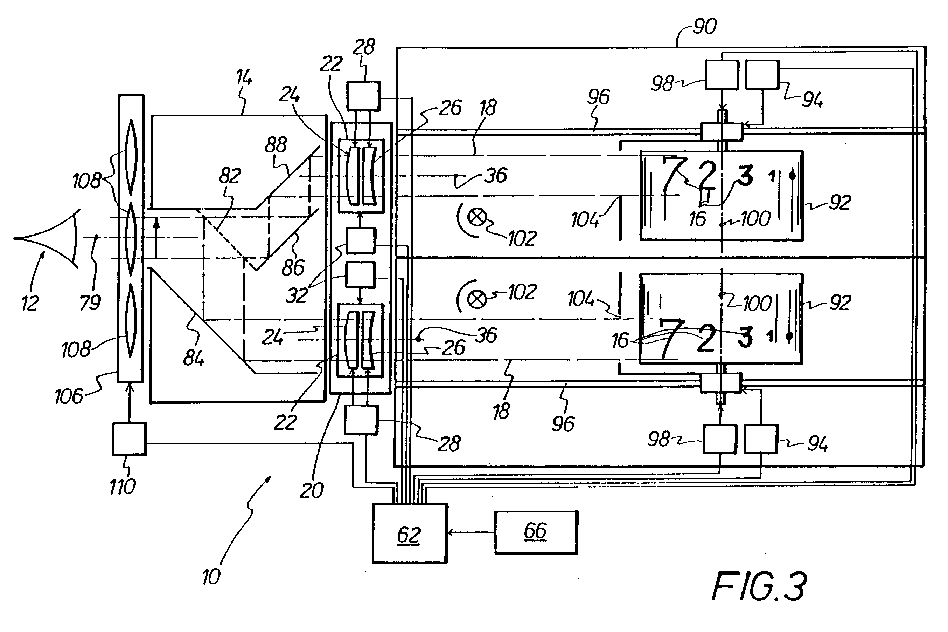

Fig. 3 zeigt schematisch eine Ausbildung des Refraktionsgerätes 10 zur simultanen Refraktionsbestimmung im Nahbereich, das eine Strahlenaufteilungseinrichtung 14 aufweist, die mit zwei hintereinander angeordneten Paaren von jeweils parallelen Spiegeln 82 und 84 bzw. 86 und 88 ausgebildet ist. Die parallelen Spiegel 82 und 84 bilden das eine Paar Spiegel und die Spiegel 86 und 88 bilden das zweite Paar Spiegel. Bei dem Spiegel 82 handelt es sich um einen halbdurchlässigen Spiegel, hinter dem der erste Spiegel 86 des zweiten Spiegelpaares mindestens annähernd senkrecht ausgerichtet vorgesehen ist. Die zweiten Spiegel 84 und 88 der beiden Spiegelpaare sind gegeneinander seitlich versetzt. Durch eine solche Ausbildung der Strahlenaufteilungeinrichtung 14 ergibt sich eine Strahlenaufteilung in zwei Teilstrahlengänge 18 für zwei Sichtzeichen 16. In jedem der beiden Teilstrahlengänge 18 ist auch bei dieser Ausbildung des Refraktionsgerätes 10 auf der vom Patienten (Auge 12) abgewandten Seite der Stahlenaufteilungseinrichtung 14 eine Optikeinrichtung 20 mit Zylinderkompensatoren 22 vorgesehen, die jeweils im zugehörigen Teilstrahlengang 18 angeordnet sind. Jeder der beiden Zylinderkompensatoren 22 weist ein Pluszylinderglas 24 und ein Minuszylinderglas 26 auf, die relativ zueinander um die zugehörige Teilstrahlengangachse 36 mittels einer ersten Antriebseinrichtung 28 verdrehbar sind, um eine gewünschte Zylinderhöhe einzustellen. Jeder Zylinderkompensator 22 ist außerdem mit einer zweiten Antriebseinrichtung 32 verbunden, um den entsprechenden Zylinderkompensator 22 insgesamt um die zugehörige Teilstrahlengangachse 36 zu verdrehen. Hierdurch ist es möglich, die entsprechende Zylinderachse einzustellen.

Auf der vom Patienten abgewandten Seite der Strahlenaufteilungseinrichtung 14 bzw. der Optikeinrichtung 20 ist eine Sehzeicheneinrichtung 90 mit zwei Sehzeichenelementen 92 vorgesehen. Hierdurch ist eine simultane Refraktionsbestimmung im Nahbereich möglich. Jedes der beiden Sehzeichenelemente ist als Trommel ausgebildet, die mittels einer sechsten Antriebseinrichtung 94 entlang einer Führungseinrichtung 96 in bezug auf die Optikeinrichtung 20 linear verstellbar ist. Jede der beiden als Trommeln ausgebildeten Sehzeichenelemente 92 weist unterschiedlich große Sehzeichen 16 auf und ist mittels einer neunten Antriebseinrichtung 98 um ihre Mittelachse 100 verdrehbar, um wunschgemäß eines der unterschiedlich großen Sehzeichen 16 in den entsprechenden Teilstrahlengang 18 hineinzustellen. Dies geschieht dazu, um das für die Sehschärfen- und Refraktionsbestimmung notwendige, kleinstmögliche noch erkennbare Sehzeichen anzubieten und dazu, die unterschiedliche Sehzeichengröße im Auge auszugleichen, die durch Näherkommen und Wegfahren des Sehzeichenschlittens auf der Netzhaut entsteht. Zur Beleuchtung der besagten Sehzeichen 16 sind Lichtquellen 102 vorgesehen. Jeder der beiden Teilstrahlengänge 18 wird mittels einer Blende 104 genau auf eines der Sehzeichen 16 des entsprechenden Sehzeichenelementes 92 gerichtet. Die Beleuchtung dient dem wunschweisen Ausblenden eines Strahlengangs. Eine weitere Funktion kommt ihr beim Anwenden einer der konventionellen Refraktionsstrategien zu, in dem die Gläser nacheinander durch Ein- bzw. Ausschalten des jeweiligen Strahlengangs angeboten werden. Auf der dem Patienten zugewandten Seite kann vor der Strahlenaufteilungeinrichtung 14 eine Bereichswählscheibe 106 mit sphärischen Gläsern 108 unterschiedlicher Dioptrienzahlen vorgesehen sein. Die sphärischen Gläser 108 können nacheinander mittels einer siebenten Antriebseinrichtung 110 im Lichtstrahl 79 der Eintrittspupille angeordnet werden. Sie dienen dem Erweitern des Meßbereiches, um z.B. +20 dptr bis -20 dptr stufenlos anbieten zu können.

Die vorzugsweise als elektrische Schrittmotoren ausgebildeten Antriebseinrichtungen 28, 32, 94, 98 und 110 sind mit einem Microcomputer 62 verbunden, der mit einer Tastatur 66 kombiniert ist. Die Wirkungsweise des Microcomputers 62 mit der Tastatur 66 ist bereits weiter oben in Verbindung mit Fig. 1 beschrieben worden.

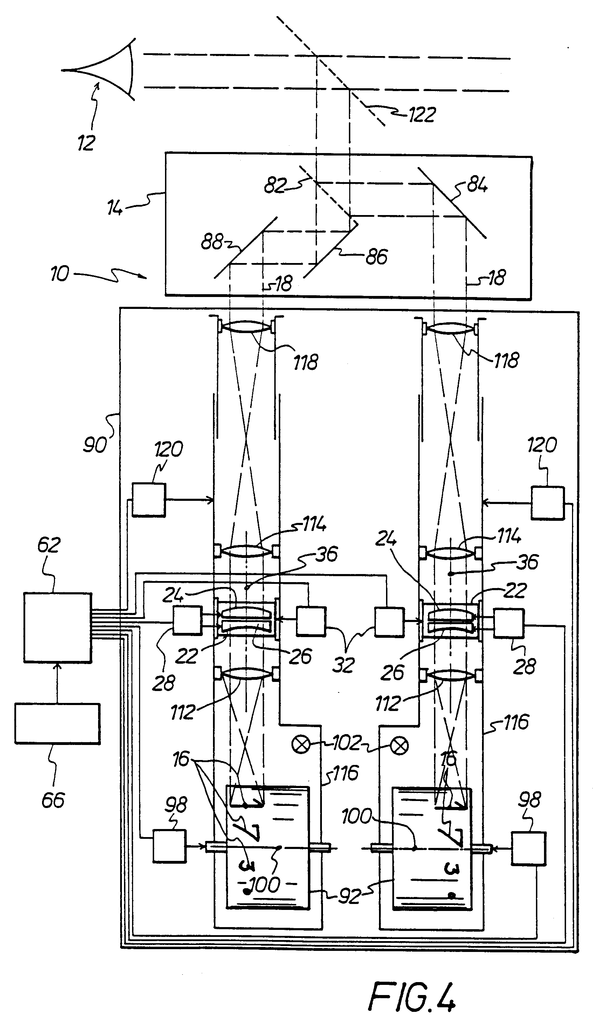

Aus Fig. 4 ist schematisch ein Refraktionsgerät 10 ersichtlich, das als Freisichtgerät zur simultanen Refraktionsbestimmung mit Sphärenbestimmung nach dem Prinzip des astronomischen Fernrohrs ausgebildet ist. Es weist eine Strahlenaufteilungseinrichtung 14 mit zwei hintereinander angeordneten Paaren von jeweils parallelen Spiegel 82, 84 und 86, 88 auf, wobei wie bei der Strahlenaufteilungseinrichtung 14 gemäß Fig. 3 der Spiegel 82 des einen Spiegelpaares als halbdurchlässiger Spiegel ausgebildet ist. Auf diese Weise ergibt sich wiederum eine Strahlenaufteilung in zwei Teilstrahlengänge 18, wobei in jedem Teilstrahlengang 18 ein Sehzeichen 16 eines Sehzeichenelementes 92 angeordnet ist. In jedem Teilstrahlengang 18 ist ein Zylinderkompensator 22 angeordnet, der ein Pluszylinderglas 24 und ein Minuszylinderglas 26 aufweist. Das Plus- und das Minuszylinderglas 24, 26 jedes Zylinderkompensators 22 sind mit einer ersten Antriebseinrichtung 28 verbunden, mit deren Hilfe es möglich ist, das Pluszylinderglas 24 und das Minuszylinderglas 26 relativ zueinander wunschgemäß um die gemeinsame Teilstrahlengangachse 36 zu verdrehen. Hierdurch ist es möglich, jede gewünschte Zylinderhöhe einzustellen. Jeder Zylinderkompensator 22 ist außerdem mit einer zugehörigen zweiten Antriebseinrichtung 32 verbunden. Mit Hilfe der zweiten Antriebseinrichtung 32 ist es möglich, den zugehörigen Zylinderkompensator 22 insgesamt um die entsprechende Teilstrahlengangachse 36 zu verdrehen, um die Zylinderachse zu bestimmen. Jeder der beiden Zylinderkompensatoren 22 ist zwischen zwei Sammellinsen 112 und 114 ortsfest vorgesehen. Das Linsensystem 114, 24, 26 und 112 jedes der beiden Teilstrahlengänge 18 ist außerdem zum zugehörigen Sehzeichenelement 92 ortsfest angeordnet. Hierdurch ist jedem der beiden Teilstrahlengänge 18 ein Fernrohrteilsystem 116 zugeordnet, das in bezug auf eine im entsprechenden Teilstrahlengang 18 ortsfest angeordnete Sammellinse 118 mit Hilfe einer zugehörigen achten Antriebseinrichtung 120 linear entlang dem besagten Teilstrahlengang 18 verstellbar ist, um die Sphäre einzustellen. Die Zylinderkompensatoren können auch zwischen Linse 118 und Strahlenteiler 14 in den Strahlengang eingeführt werden. Das Fernrohrsystem könnte auch durch Bewegen der Linse 114 in Richtung des Strahlenganges ersetzt werden.

Jedes der beiden als Trommeln ausgebildeten Sehzeichenelemente 92 mit den unterschiedlich großen Sehzeichen 16 ist mit einer neunten Antriebseinrichtung 98 verbunden, mit deren Hilfe es möglich ist, das zugehörige Sehzeichenelement 92 um seine Mittelachse 100 wunschgemäß zu verdrehen. Zur Beleuchtung der Sehzeichen 16 sind auch bei dieser Ausbildung des Refraktionsgerätes 10 in der Sehzeicheneinrichtung 90 Lichtquellen 102 vorhanden.

Die Antriebseinrichtungen 28, 32, 98 und 120, die vorzugsweise als Schrittmotoren ausgebildet sind, sind mit einem Microcomputer 62 zusammengeschaltet, der mit einer Tastatur 66 verbunden ist. Die Wirkungsweise der zuletzt genannten Elemente ist bereits weiter oben beschrieben worden, so daß es sich erübrigt, diese noch einmal im Detail zu beschreiben.

Auf der dem Patienten (Auge 12) zugewandten Seite der Strahlenaufteilungseinrichtugn 14 des Refraktionsgerätes 10 gemäß Fig. 4 ist zur Ausbildung eines Freisichtgerätes ein halbdurchlässiger Spiegel 122 vorgesehen.

## Patentansprüche

1. Refraktionsgerät zur subjektiven Bestimmung der sphärischen und astigmatischen Seheigenschaften des Auges (12) eines Patienten mit einer Einrichtung (14) zur Strahlenaufteilung in zwei Teilstrahlengänge (18), wodurch mindestens ein Sehzeichen (16) im Auge (12) in zwei mit einem Blick erfaßbare Vergleichsbilder (16') aufgeteilt wird, und mit einer dem jeweiligen Teilstrahlengang (18) zugeordneten Optikeinrichtung (20), die zur Einstellung bestimmter optischer Eigenschaften in den beiden Teilstrahlengänge (18) vorgesehen ist,
**dadurch gekennzeichnet,**
daß in jedem der beiden Teilstrahlengänge (18) als Optikeinrichtung (20) ein Zylinderkompensator (22) angeordnet ist, der mindestens ein Pluszylinderglas (24) und mindestens ein Minuszylinderglas (26) aufweist, wobei das/jedes im entsprechenden Teilstrahlengang (18) vorgesehene Plus- und Minuszylinderglas (24, 26) zur Zylinderhöheneinstellung mittels einer zugehörigen ersten Antriebseinrichtung (28) gegeneinander um eine mit der entsprechenden Teilstrahlengangachse (36) zusammenfallende Drehachse verdrehbar sind, und wobei das/jedes Plus- und das/jedes Minuszylinderglas (24, 26) jedes Zylinderkompensators (22) zur entsprechenden Zylinderachseneinstellung mittels einer zugehörigen zweiten Antriebseinrichtung (32) um die entsprechende Teilstrahlengangachse (36) jeweils gemeinsam verdrehbar sind.

2. Refraktionsgerät nach Anspruch 1,
**dadurch gekennzeichnet,**
daß die Strahlenaufteilungseinrichtung (14) zwei Paare von jeweils zueinander mindestens annähernd parallelen Spiegeln (38, 40; 42, 44) aufweist, die von der Patientenseite weg divergieren, wobei die Spiegelpaare (38, 40; 42, 44) zueinander mindestens annähernd senkrecht orientiert sind.

3. Refraktionsgerät nach Anspruch 2,
**dadurch gekennzeichnet,**
daß das eine Paar Spiegel (38, 40) ortsfest und daß ein Spiegel (44) des zweiten Paares Spiegel (42, 44) zur Ausrichtung der beiden Teilstrahlengänge (18) auf ein einziges Sichtzeichen (16) mittels einer dritten Antriebseinrichtung (48) in seinem Winkel relativ zum anderen Spiegel (42) des zweiten Paares Spiegel (42, 44) verstellbar ist.

4. Refraktionsgerät nach einem der vorhergehenden Ansprüche,
**dadurch gekennzeichnet,**
daß zum Sphäre-Vergleich eine mit sphärischen Gläsern (54) und mit einem Verschluß versehene Scheibe (52) vorgesehen ist, die auf der vom Patienten abgewandten Seite nach den beiden Zylinderkompensatoren (22) angeordnet und mittels einer vierten Antriebseinrichtung (56) um eine zu den beiden Teilstrahlengängen (18) parallele Drehachse drehbar ist.

5. Refraktionsgerät nach einem der vorhergehenden Ansprüche,
**gekennzeichnet durch**
einen auf der dem Patienten zugewandten Seite vorgesehenen, eine Geräteeintrittspupille bildenden Montageabschnitt (60) zur Befestigung des Refraktionsgerätes (10) als Vorsatzgerät an einem an sich bekannten Phoropter.

6. Refraktionsgerät nach Anspruch 5,
**dadurch gekennzeichnet,**
daß auf der dem Patienten zugewandten Seite der Geräteeintrittspupille zur Ausbildung eines Gerätes für eine 5-Meter-Visus-Strecke zwei zueinander parallele Scheiben (70, 72) mit im gemeinsamen Strahlengang (79) vor der Geräteeintrittspupille anordenbaren sphärischen Gläsern (74, 76) vorgesehen sind, wobei die sphärischen Gläser (74) der einen Scheibe (70) einen im Vergleich zum Dioptrienabstand der sphärischen Gläsern (76) der zweiten Scheibe (72) großen Dioptrienabstand aufweisen, und die beiden Scheiben (70, 72) mittels einer fünften Antriebseinrichtung (78) wunschgemäß verdrehbar sind.

7. Refraktionsgerät nach Anspruch 1,
**dadurch gekennzeichnet,**
daß die Einrichtung (14) zur Strahlenaufteilung zwei hintereinander angeordnete Paare (82, 84; 86, 88) von jeweils parallelen Spiegeln (82, 84; 86, 88) aufweist, wobei der eine Spiegel (82) des einen Spiegelpaares (82, 84) ein halbdurchlässiger Spiegel ist, hinter dem der erste Spiegel (86) des zweiten Spiegelpaares (86, 88) zum halbdurchlässigen Spiegel (82) des ersten Spiegelpaares (82, 84) mindestens annähernd senkrecht ausgerichtet ist und die zweiten Spiegel (84, 88) der beiden Spiegelpaare gegeneinander seitlich versetzt sind.

8. Refraktionsgerät nach Anspruch 7,
**dadurch gekennzeichnet,**
daß auf der vom Patienten abgewandten Seite der Strahlenaufteilungseinrichtung (14) zur simultanen Refraktionsbestimmung im Nahbereich eine Sehzeicheneinrichtung (90) mit zwei Sehzeichenelementen (92) vorgesehen ist, von welchen jedes in einem zugehörigen Teilstrahlengang (18) angeordnet und mittels einer zugehörigen sechsten Antriebseinrichtung (94) in bezug auf die Strahlenaufteilungseinrichtung (14) linear verstellbar ist.

9. Refraktionsgerät nach Anspruch 7 und 8,
**dadurch gekennzeichnet,**
daß in jedem Teilstrahlengang (18) der entsprechende, mit der ersten und der zweiten Antriebseinrichtung (28, 32) verbundene Zylinderkompensator (22) zwischen der Strahlenaufteilungseinrichtung (14) und der Sehzeicheneinrichtung (90) ortsunveränderlich angeordnet ist, und daß auf der dem Patienten zugewandten Seite vor der Strahlenaufteilungseinrichtung (14) eine Bereichswählscheibe (106) mit sphärischen Gläsern (108) unterschiedlicher Dioptrienzahl vorgesehen ist, die wahlweise nacheinander mittels einer siebenten Antriebseinrichtung (110) im Lichtstrahl (79) der Eintrittspupille (79) anordenbar bzw. austauschbar sind.

10. Refraktionsgerät nach Anspruch 7 und 8,
**dadurch gekennzeichnet,**
daß in jedem Teilstrahlengang (18) der zugehörige, mit der ersten und der zweiten Antriebseinrichtung (28, 32) verbundene Zylinderkompensator (22) zwischen zwei Sammellinsen (112, 114) und zu diesen und in bezug auf das zugehörige Sehzeichenelement (92) in einem entsprechenden Fernrohrteilsystem (116) ortsunveränderlich vorgesehen ist, und daß jedes der beiden Fernrohrteilsysteme (116) mittels einer zugehörigen achten Antriebseinrichtung (120) linear entlang dem entsprechenden Teilstrahlengang (18) relativ zu einer im entsprechenden Teilstrahlengang (18) auf der vom Patienten abgewandten Seite der Strahlenaufteilungseinrichtung (14) ortsfest angeordneten Sammellinse (118) verstellbar ist.

11. Refraktionsgerät nach Anspruch 10,
**dadurch gekennzeichnet,**
daß auf der dem Patienten zugewandten Seite der Strahlenaufteilungseinrichtung (14) zur Ausbildung eines Freisichtgerätes ein halbdurchlässiger Spiegel (122) schräg orientiert vorgesehen ist.

12. Refraktionsgerät nach einem der Ansprüche 8 bis 11,
**dadurch gekennzeichnet,**
daß jede der beiden Sehzeicheneinrichtungen (92) unterschiedlich große Sehzeichen (16) aufweist und mittels einer zugehörigen neunten Antriebseinrichtung (98) relativ zum entsprechenden Teilstrahlengang (18) verstellbar ist.

13. Refraktionsgerät nach einem der vorhergehenden Ansprüche,
**dadurch gekennzeichnet,**
daß die beiden ersten Antriebseinrichtungen (28) und/oder die beiden zweiten Antriebseinrichtungen (32) und/oder die dritte Antriebseinrichtung (48) und/oder die vierte Antriebseinrichtung (56) und/oder die fünfte Antriebseinrichtung (78) und/oder beiden sechsten Antriebseinrichtungen (94) und/oder die siebente Antriebseinrichtung (110) und/oder die beiden achten Antriebseinrichtungen (120) und/oder die beiden neunten Antriebseinrichtungen (98) mit einem Microcomputer (62) verbundene Schrittmotoren sind.

## Claims

1. A refraction device for the subjective determination of the spherical and astigmatic sight properties of the eye (12) of a patient, having a device (14) for dividing a beam into two partial beam paths (18) whereby at least one sight character (16) is divided in the eye (12) into two comparative images (16') which can be perceived with a look, and an optical device (20) which is associated with the respective partial beam path (18) and which is provided for setting given optical properties in the two partial beam paths (18) characterised in that disposed in each of the two partial beam paths (18) as the optical device (20) is a cylindrical compensator (22) which has at least one positive cylindrical lens (24) and at least one negative cylindrical lens (26), wherein the/each positive and negative cylindrical lens (24, 26) provided in the corresponding partial beam path (18) are rotatable relative to each other by means of an associated first drive device (28) about an axis of rotation which is coincident with the corresponding partial bed path axis (36) for cylinder height adjustment and wherein the/each positive and the/each negative cylindrical lens (24, 26) of each cylindrical compensator (22) are respectively jointly rotatable by means of an associated second drive device (32) about the corresponding partial beam path axis (36) for corresponding cylinder axis adjustment.

2. A refraction device as set forth in claim 1 characterised in that the beam dividing device (14) has two pairs of mirrors (38, 40; 42, 44) which are respectively at least approximately parallel to each other and which diverge away from the patient side, the pairs of mirrors (38, 40; 42, 44) being at least approximately perpendicularly oriented relative to each other.

3. A refraction device as set forth in claim 2 characterised in that the one pair of mirrors (38, 40) is stationary and that a mirror (44) of the second pair of mirrors (42, 44) is adjustable in respect of its angle relative to the other mirror (42) of the second pair of mirrors (42, 44) by means of a third drive device (48) for alignment of the two partial beam paths (18) with a single sight character (16).

4. A refraction device as set forth in one of the preceding claims characterised in that for spherical comparison there is provided a disk (52) which is provided with spherical lenses (54) and with a shutter and which is arranged on the side remote from the patient after the two cylindrical compensators (22) and which is rotatable by means of a fourth drive device (56) about an axis of rotation that is parallel to the two partial beam paths (18).

5. A refraction device as set forth in one of the preceding claims characterised by a mounting portion (60) which is disposed on the side towards the patient and which forms a device entrance pupil, for fixing the refraction device (10) in the form of a front accessory device on a per se known phoropter.

6. A refraction device as set forth in claim 5 characterised in that disposed on the side of the device entrance pupil that is towards the patient, for forming a device for a 5-meter sight distance, are two mutually parallel disks (70, 72) with spherical lenses (74, 76) which can be arranged in the common beam path (79) in front of the device entrance pupil, wherein the spherical lenses (74) of the one disk (70) have a diopter interval which is large in comparison with that of the spherical lenses (76) of the second disk (72), and the two disks (70, 72) are rotatable as desired by means of a fifth drive device (78).

7. A refraction device as set forth in claim 1 characterised in that the beam dividing device (14) has two successively arranged pairs (82, 84; 86, 88) of respectively parallel mirrors (82, 84; 86, 88), wherein the one mirror (82) of the one pair of mirrors (82, 84) is a semi-transparent mirror, behind which the first mirror (86) of the second pair of mirrors (86, 88) is oriented at least approximately perpendicularly to the semi-transparent mirror (82) of the first pair of mirrors (82, 84) and the second mirrors (84, 88) of the two pairs of mirrors are laterally displaced relative to each other.

8. A refraction device as set forth in claim 7 characterised in that disposed on the side of the beam dividing device (14) that is remote from the patient for simultaneous refraction determination in the near region is a sight character device (90) with two sight character elements (92), each of which is arranged in an associated partial beam path (18) and is linearly displaceable by means of an associated sixth drive device (94) in relation to the beam dividing device (14).

9. A refraction device as set forth in claim 7 and claim 8 characterised in that in each partial beam path (18) the correspondingly cylindrical compensator (22) which is connected to the first and second drive devices (28, 32) is arranged to be invariable in respect of position between the beam dividing device (14) and the sight character device (90) and that on the side towards the patient in front of the beam dividing device (14) is a range selector disk (106) with spherical lenses (108) of different diopter numbers which can be selectively successively interchanged or arranged in the light beam (79) of the entrance pupil (79) by means of a seventh drive device (110).

10. A refraction device as set forth in claims 7 and 8 characterised in that in each partial beam path (18) the associated cylindrical compensator (22) which is connected to the first and second drive devices (28, 32) is disposed between two converging lenses (112, 114) invariably in respect of position relative to same and in relation to the associated sight character element (92), in a corresponding telescope sub-system (116), and that each of the two telescope sub-systems (116) is adjustable by means of an associated eighth drive device (120) linearly along the corresponding partial beam path (18) relative to a converging lens (118) which is arranged stationarily in the corresponding partial beam path (18) on the side of the beam dividing device (14), that is remote from the patient.

11. A refraction device as set forth in claim 10 characterised in that a semi-transparent mirror (122) is disposed in an inclined orientation on the side of the beam dividing device (14), that is towards the patient, to provide a free-sight device.

12. A refraction device as set forth in one of claims 8 to 11 characterised in that each of the two sight character devices (92) has sight characters (16) of different sizes and is adjustable by means of an associated ninth drive device (98) relative to the corresponding partial beam path (18).

13. A refraction device as set forth in one of the preceding claims characterised in that the two first drive devices (28) and/or the two second drive devices (32) and/or the third drive device (48) and/or the fourth drive device (56) and/or the fifth drive device (78) and/or both sixth drive devices (94) and/or the seventh drive device (110) and/or the two eighth drive devices (120) and/or the two ninth drive devices (98) are stepping motors connected to a microcomputer (62).

## Revendications

1. Dispositif à réfraction, pour la détermination subjective de particularités visuelles sphériques et astigmatiques de l'oeil (12) d'un patient, comportant un équipement (14) pour le partage de rayons en deux parcours de rayon partiel (18), et par cela au moins une mire (16) est partagée dans l'oeil (12) en deux images de comparaison (16') qui peuvent être saisies en un coup d'oeil, et comportant un équipement optique (20) qui est adjoint à chaque parcours de rayon partiel (18) et qui est prévu pour le réglage de particularités optiques dans les deux parcours de rayon partiel (18), caractérisé en ce que dans chacun des deux parcours de rayon partiel (18) est agencé en tant qu'équipement optique (20) un compensateur cylindrique qui présente au moins un verre cylindrique positif (24) et au moins un verre cylindrique négatif (28), le ou chaque verre cylindrique positif ou négatif (24, 26) prévu dans le parcours de rayon partiel correspondant pouvant être tourné par rapport à l'autre autour d'un axe de rotation, qui coïncide avec l'axe de parcours de rayon partiel (36) correspondant, pour le réglage en hauteur du cylindre au moyen d'un premier équipement d'entraînement (28) correspondant, et le ou chaque verre cylindrique positif et le ou chaque verre cylindrique négatif (24, 26) de chaque compensateur cylindrique (22) pouvant être tourné chaque fois ensemble autour de l'axe de parcours de rayon partiel (36) correspondant pour le réglage de l'axe de cylindre correspondant au moyen d'un second équipement d'entraînement (32) correspondant.

2. Dispositif à réfraction suivant la revendication 1, caractérisé en ce que l'équipement de partage de rayons (14) présente deux paires de miroirs (38, 40; 42, 44) chaque fois au moins approximativement parallèles l'un à l'autre et qui divergent à partir du côté du patient, les paires de miroirs (38, 40; 42, 44) étant orientées au moins approximativement perpendiculairement l'une à l'autre.

3. Dispositif à réfraction suivant la revendication 2, caractérisé en ce qu'une paire de miroirs (38, 40) est fixe et en ce qu'un miroir (44) de la seconde paire de miroirs (42, 44) est réglable, selon son angle par rapport à l'autre miroir (42) de la seconde paire de miroirs (42, 44), au moyen d'un troisième équipement d'entraînement (48) pour l'orientation des deux parcours de rayon partiel (18) sur une mire unique (16).

4. Dispositif à réfraction suivant l'une des revendications précédentes, caractérisé en ce que pour la comparaison sphérique, il est prévu une plaque (52) qui est équipée de verres sphériques (54) et d'un obturateur, qui est agencée à la suite des deux compensateurs cylindriques (22), du côté tourné à l'écart du patient, et qui peut être tournée autour d'un axe de rotation parallèle aux deux parcours de rayon partiel (18) au moyen d'un quatrième équipement d'entraînement (56).

5. Dispositif à réfraction suivant l'une des revendications précédentes, caractérisé par un tronçon de montage (60) qui est prévu du côté tourné vers le patient, qui forme une pupille d'entrée du dispositif et qui est destiné à la fixation du dispositif à réfraction (10), en tant qu'adaptateur, sur un ophtalmomètre connu en soi.

6. Dispositif à réfraction suivant la revendication 5, caractérisé en ce que pour la réalisation d'un dispositif pour une distance de vision de 5 m, deux plaques (70, 72) parallèles l'une à l'autre et comportant dans le parcours de rayon commun (79) des verres sphériques (74, 76) qui peuvent être agencés avant la pupille d'entrée du dispositif sont prévues sur le côté, tourné vers le patient, de la pupille d'entrée du dispositif, les verres sphériques (74) d'une des plaques (70) présentant une grande distance dioptrique en comparaison à la distance dioptrique des verres sphériques (76) de la seconde plaque (72), et en ce que les deux plaques (70, 72) peuvent être tournées à volonté au moyen d'un cinquième équipement d'entraînement (78).

7. Dispositif à réfraction suivant la revendication 1, caractérisé en ce que l'équipement (14) pour le partage de rayons présente deux paires (82, 84; 86, 88), agencées l'une derrière l'autre, de miroirs (82, 84; 86, 88) respectivement parallèles, un des miroirs (82) d'une des paires de miroirs (82, 84) étant un miroir semi-transparent derrière lequel le premier miroir (86) de la seconde paire de miroirs (86, 88) est orienté au moins approximativement perpendiculairement par rapport au miroir semi-transparent (82) de la première paire de miroirs (82, 84), les seconds miroirs (84, 88) des deux paires de miroirs étant décalés latéralement l'une par rapport à l'autre.

8. Dispositif à réfraction suivant la revendication 7, caractérisé en ce que du côté tourné à l'écart du patient de l'équipement de partage de rayons (14), il est prévu pour la détermination de réfraction simultanée dans la zone proche un équipement de mire (90) comportant deux éléments de mire (92) dont chacun est agencé dans un parcours de rayon partiel (18) correspondant et peut être réglé linéairement, au moyen d'un sixième équipement d'entraînement (94) correspondant, par rapport à l'équipement de partage de rayons (14).

9. Dispositif à réfraction suivant la revendication 7 ou 8, caractérisé en ce que dans chaque parcours de rayon partiel (18), le compensateur cylindrique (22) relié au premier et au second équipement d'entraînement (28, 32) est agencé de façon non modifiable en position, entre l'équipement de partage de rayons (14) et l'équipement de mire (90) et en ce que, du côté tourné vers le patient, il est prévu avant l'équipement de partage de rayons (14) une plaque de sélection de zone (106) comportant des verres sphériques (108) de nombre de dioptrie différent qui peuvent être agencés ou échangés sélectivement l'un après l'autre, au moyen d'un septième équipement d'entraînement (110), dans le rayon lumineux (79) de la pupille d'entrée (79).

10. Dispositif à réfraction suivant la revendication 7 ou 8, caractérisé en ce que, dans chaque parcours de rayon partiel (18), le compensateur cylindrique (22) correspondant relié au premier et au second équipement d'entraînement (28, 32) est prévu entre deux lentilles de concentration (112, 114) et de façon non modifiable en position par rapport à celles-ci et par rapport à l'élément de mire (92) correspondant, dans un système partiel de télescope (116) correspondant et en ce que chacun des deux systèmes partiels de télescope (116) peut être réglé linéairement, au moyen d'un huitième équipement d'entraînement (120) correspondant, le long du parcours de rayon partiel (18) correspondant, par rapport à une lentille de concentration (118) agencée de façon fixe en position dans le parcours de rayon partiel (18) correspondant, du côté tourné à l'écart du patient de l'équipement de partage de rayons (14).

11. Dispositif à réfraction suivant la revendication 10, caractérisé en ce que, du côté tourné du côté du patient de l'équipement de partage des rayons (14), il est prévu de façon orientée en oblique un miroir semi-transparent (122) pour la réalisation d'un dispositif à vue libre.

12. Dispositif à réfraction suivant l'une des revendications 8 à 11, caractérisé en ce que chacun des deux équipements de mire (92) présente des mires (16) de grandeurs différentes et est réglable par rapport au parcours de rayon partiel (18) correspondant au moyen d'un neuvième équipement d'entraînement (98) correspondant.

13. Dispositif à réfraction suivant l'une des revendications précédentes, caractérisé en ce que les deux premiers équipements d'entraînement (28) et/ou les deux seconds équipements d'entraînement (32) et/ou le troisième équipement d'entraînement (48) et/ou le quatrième équipement d'entraînement (56) et/ou le cinquième équipement d'entraînement (78) et/ou les deux sixièmes équipements d'entraînement (94) et/ou le septième équipement d'entraînement (110) et/ou les deux huitièmes équipements d'entraînement (120) et/ou les deux neuvièmes équipements d'entraînement (98) sont des moteurs pas-à-pas reliés à un micro-ordinateur (62).
